# EUROPEAN PATENT APPLICATION

(11) **EP 4 826 368 A1**
(43) Date of publication of application: **21.10.2026**
(21) Application number: 24903447.1
(22) Date of filing: 19.11.2024
(51) Int. Cl.: G01N 27/02, G01N 33/48, G01N 33/483

(54) **SUBSTRATE AND INSPECTION SYSTEM**

(30) Priority: 15.12.2023 JP 2023212410
(71) Applicant: PHC HOLDINGS CORPORATION, Tokyo 100-8403 (JP)
(72) Inventor: OCHI, Shinya, Ehime 791-0395 (JP); FUTAGAMI, Norimichi, Ehime 791-0395 (JP); HARUTA, Kengo, Ehime 791-0395 (JP); KITANE, Ryoichi, Ehime 791-0395 (JP); KOJIMA, Shinki, Ehime 791-0395 (JP)
(74) Representative: Eisenführ Speiser
(86) International application number: PCT/JP2024/041035
(87) International publication number: WO 2025/126788

(57) **Abstract**

A substrate according to the present invention includes: a substrate main body; a plurality of land portions that have a sensing portion and a terminal portion and that are supported by the substrate main body; and a dummy pad portion provided in a second region surrounding a first region in which the sensing portion is provided on the substrate main body.

## Description

### Technical Field

The present invention relates to a substrate and an inspection system.

### Background Art

Patent Literature (hereinafter, referred to as PTL) 1 discloses a substrate including a substrate main body and a metal land portion provided on a surface of the substrate main body. Such a substrate is used, for example, in an inspection for determining whether cells collected from a patient with suspected skin cancer contain cancer cells.

### Citation List

### Patent Literature

PTL 1
Japanese Unexamined Patent Application Publication (Translation of PCT Application) No. 2021-516336

### Summary of Invention

### Technical Problem

Incidentally, in the case of a substrate as described above, the land portion provided on the surface of the substrate main body is subjected to a surface treatment such as plating, whereby a sensing portion is formed. It is desirable that the plating thickness dimension of such a sensing portion varies as little as possible depending on the location.

The present invention has been made in view of such circumstances, and an object of the present invention is to provide a substrate and an inspection system in which variation in the thickness dimension of the sensing portion is small.

### Solution to Problem

A substrate according to the present invention includes: a substrate main body; a plurality of land portions that each include a sensing portion and a terminal portion and are supported by the substrate main body; and a dummy pad portion provided in a certain region surrounding a first region in which the sensing portion is provided in the substrate main body, the certain region being referred to as a second region.

An inspection system according to the present invention includes: the above-described substrate; and an inspection apparatus that includes a substrate support portion for supporting the substrate and measures impedance of a target cell placed on the sensing portion of the substrate.

### Advantageous Effects of Invention

According to the present invention, it is possible to provide a substrate and an inspection system in which variation in the thickness dimension of the sensing portions is small.

### Brief Description of Drawings

[FIG. 1A] FIG. 1A is a perspective view of an inspection system according to an embodiment of the present invention;
[FIG. 1B] FIG. 1B is a schematic cross-sectional view of an inspection apparatus;
[FIG. 2] FIG. 2 is a plan view of a substrate;
[FIG. 3] FIG. 3 is a bottom view of the substrate;
[FIG. 4] FIG. 4 is a cross-sectional view of the substrate illustrated in FIG. 2 taken along line C1-C1, and is a cross-sectional view for describing the configuration of the land portion;
[FIG. 5] FIG. 5 is a cross-sectional view of the substrate illustrated in FIG. 2 taken along line C1-C1, and is a cross-sectional view for describing the configuration of the dummy pad portion;
[FIG. 6A] FIG. 6A is a plan view showing an example of a variation of the substrate;
[FIG. 6B] FIG. 6B is a plan view showing an example of a variation of the substrate;
[FIG. 6C] FIG. 6C is a plan view showing an example of a variation of the substrate;
[FIG. 6D] FIG. 6D is a plan view showing an example of a variation of the substrate;
[FIG. 6E] FIG. 6E is a plan view showing an example of a variation of the substrate;
[FIG. 6F] FIG. 6F is a plan view showing an example of a variation of the substrate;
[FIG. 6G] FIG. 6G is a plan view showing an example of a variation of the substrate;
[FIG. 7] FIG. 7 is a schematic plan view showing an example of a final work-size substrate;
[FIG. 8] FIG. 8 is a line graph showing test results;
[FIG. 9] FIG. 9 is a line graph showing test results;
[FIG. 10] FIG. 10 is a line graph showing test results;
[FIG. 11] FIG. 11 is a line graph showing test results;
[FIG. 12] FIG. 12 is a line graph showing test results; and
[FIG. 13] FIG. 13 is a line graph showing test results.

### Description of Embodiments

Hereinafter, embodiments of a substrate according to the present invention will be described with reference to the drawings. The same components are denoted by the same reference numerals. The matters described below together with the accompanying drawings are intended to describe an exemplary embodiment and are not intended to represent the sole embodiment.

### [Embodiment]

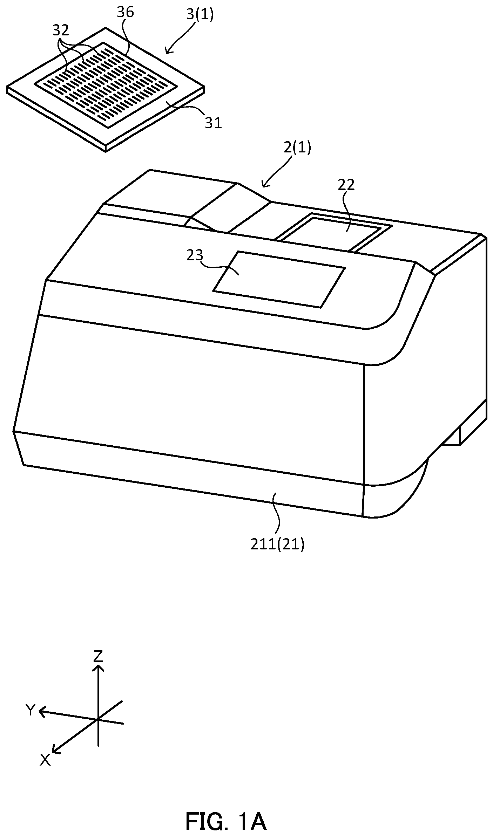

Inspection system 1 and substrate 3 according to an embodiment of the present invention will be described with reference to FIGS. 1A to 5.

FIG. 1A is a perspective view of inspection system 1 according to the embodiment of the present invention. Inspection system 1 is an inspection system for cells. Examples of the cells include various cancer cells. The cells to be inspected by inspection system 1 are referred to as target cells.

First, an outline of inspection system 1 will be described. Inspection system 1 is used, for example, during surgery to test whether cells (that is, target cells) collected from a patient by a physician are cancer cells.

An operator places target cells on an upper surface of substrate 3 set in substrate support portion 22 of inspection apparatus 2. Then, inspection apparatus 2 measures the impedance of the target cells placed on the upper surface of substrate 3. Then, inspection apparatus 2 determines whether the target cells are cancer cells based on the measured impedance. Inspection apparatus 2 displays the determination result on display portion 23.

With such inspection system 1, a physician can determine the status of the surgery while confirming the determination result of inspection apparatus 2. Therefore, the time required for the surgery can be significantly shortened.

In particular, substrate 3 according to the present embodiment includes dummy pad portion 36 to be described later. Such dummy pad portion 36 can reduce variation in the plating thickness dimension of sensing portions 34 (in other words, land portions 32) provided on the upper surface of substrate 3. As a result, with inspection system 1 including substrate 3 according to the present embodiment, variation in detection values of the impedance of the target cells can be suppressed. Thus, the reliability of the detection values can be improved by inspection system 1.

Hereinafter, first, a specific configuration of inspection system 1 according to the present embodiment will be described. Thereafter, examples of the present invention and results of tests conducted by the inventors to confirm the functions and effects of the present invention will be described.

### (Inspection System)

Inspection system 1 includes inspection apparatus 2 and substrate 3.

In the following description, in describing the structure of each member constituting inspection system 1, an orthogonal coordinate system (X, Y, Z) shown in each drawing may be used. The X direction coincides with the front-rear direction of inspection apparatus 2 constituting inspection system 1.

The X direction positive side coincides with the front side of inspection apparatus 2. The front side of inspection apparatus 2 is the side facing a user during use. The X direction negative side coincides with the rear side of inspection apparatus 2.

The Y direction coincides with the left-right direction of inspection apparatus 2. The Y direction positive side coincides with the left side when inspection apparatus 2 is viewed from the front of inspection apparatus 2. The Y direction negative side coincides with the right side when inspection apparatus 2 is viewed from the front of inspection apparatus 2.

The Z direction coincides with the up-down direction of inspection apparatus 2. The Z direction positive side coincides with the upper side of inspection apparatus 2. The upper side of inspection apparatus 2 is the side away from the ground surface on which inspection apparatus 2 is placed. The Z direction negative side coincides with the lower side of inspection apparatus 2.

### (Inspection Apparatus)

Inspection apparatus 2 corresponds to an example of a measurement apparatus and has a function of measuring the impedance of target cells. Cells have a unique impedance depending on their type. Inspection apparatus 2 identifies the type of target cells (e.g., whether the target cells are cancer cells) based on the measured impedance.

Inspection apparatus 2 includes apparatus main body 21, substrate support portion 22, and display portion 23.

Apparatus main body 21 is configured with housing 211 having a box shape and various devices (not shown) housed in housing 211. The devices include, for example, a main board (not shown) and a control section (not shown). The control section is mounted on the main board and controls the overall operation of inspection apparatus 2.

### (Substrate support portion)

Substrate support portion 22 is provided on the upper surface of housing 211. Substrate support portion 22 is a portion that supports substrate 3 during use of inspection apparatus 2 (that is, during inspection).

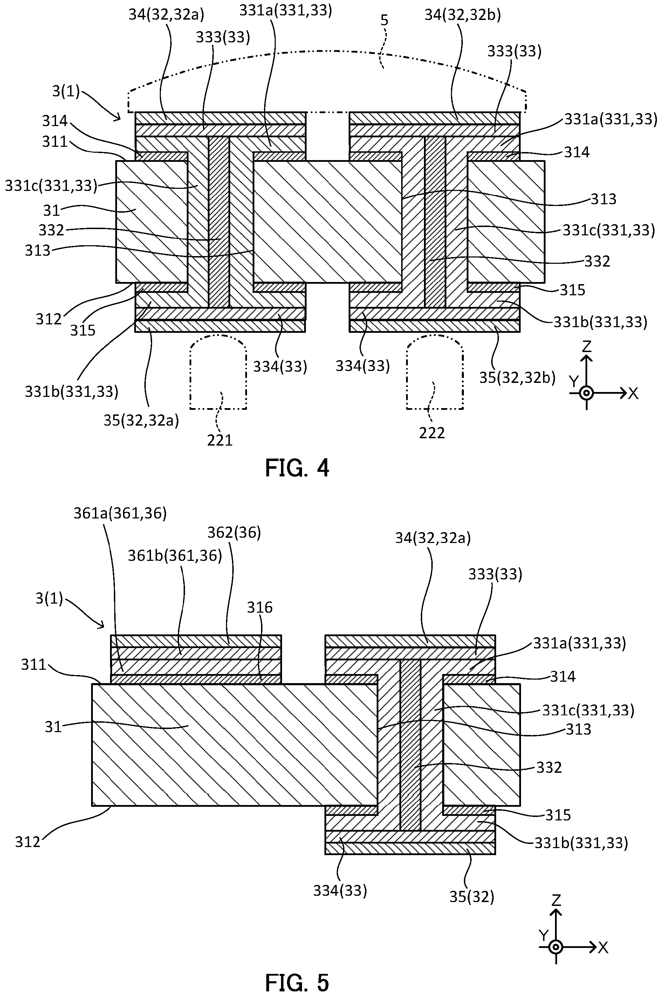

Substrate support portion 22 is provided, for example, on the upper surface of housing 211. Substrate support portion 22 has inspection region 22R provided on the upper surface of housing 211. Inspection region 22R is a space that opens upward. Further, substrate support portion 22 includes at least a pair of apparatus-side terminal portions 221 and 222 (see FIG. 4) in inspection region 22R.

Apparatus-side terminal portions 221 and 222 each have a pin shape extending in the vertical direction. During use, each of apparatus-side terminal portions 221 and 222 comes into contact with terminal portion 35 of land portion 32 in substrate 3 to be described later.

Apparatus-side terminal portion 221 is connected to the positive electrode of the power supply via an electric circuit (not shown) provided in inspection apparatus 2. Apparatus-side terminal portion 222 is connected to the negative electrode of the power supply via an electric circuit (not shown) provided in inspection apparatus 2.

Furthermore, substrate support portion 22 includes mounting portion 223 on which the outer edge portion of substrate 3 is mounted in inspection region 22R. Substrate support portion 22 includes positioning member 224 at mounting portion 223. Positioning member 224 engages with through-hole 31a provided in the outer edge portion of substrate 3 to position substrate 3. In addition, substrate support portion 22 includes cover 225 that covers inspection region 22R.

When substrate 3 is placed on mounting portion 223 of substrate support portion 22, apparatus-side terminal portions 221 and 222 come into contact with terminal portion 35 of land portion 32 of substrate 3 from below.

### (Display portion)

Display portion 23 is provided on the front surface of housing 211. Display portion 23 is a display that displays information. During inspection, display portion 23 displays the impedance of the target cells measured by inspection apparatus 2 and/or the type of the target cells identified based on the impedance of the target cells measured by inspection apparatus 2 (whether the target cells are cancer cells, and the like).

### (Substrate)

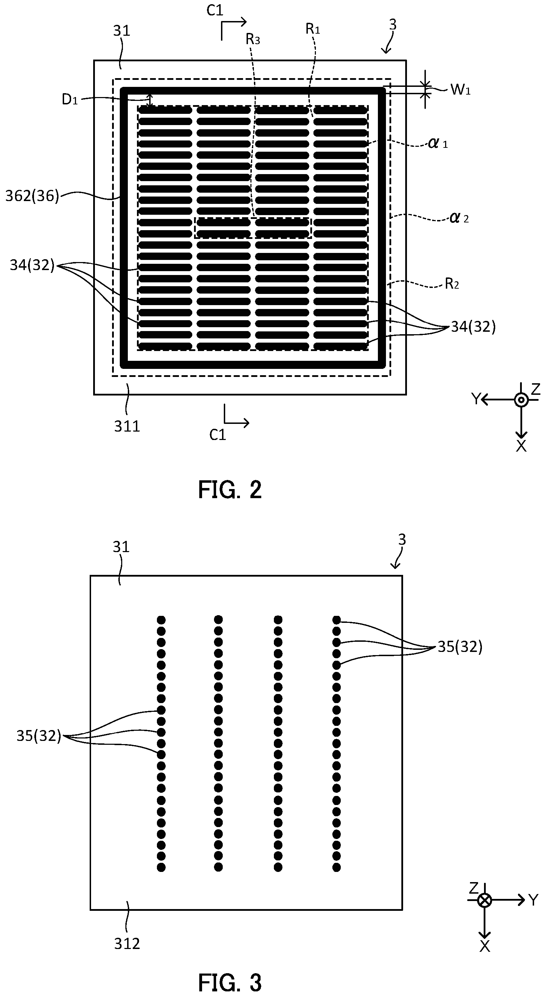

Next, substrate 3 will be described with reference to FIGS. 1A to 5. FIG. 2 is a plan view of substrate 3. FIG. 3 is a bottom view of substrate 3. FIG. 4 is a cross-sectional view of substrate 3 taken along line C1-C1 in FIG. 2, and is a cross-sectional view for describing the configuration of land portion 32. In FIG. 4, only two adjacent land portions 32 are illustrated. FIG. 5 is a cross-sectional view of substrate 3 taken along line C1-C1 in FIG. 2, and is a cross-sectional view for describing the configuration of dummy pad portion 36.

Substrate 3 includes substrate main body 31, a plurality of land portions 32, and dummy pad portion 36. Substrate 3 is placed on substrate support portion 22 (see FIG. 1A) of inspection apparatus 2 during use (in other words, during inspection).

### (Substrate Main Body)

Substrate main body 31 is a rectangular plate-shaped body made of a resin. Substrate main body 31 includes first surface 311 (in other words, upper surface) disposed on the upper side and second surface 312 (in other words, lower surface) disposed on the lower side during use. Second surface 312 faces substrate support portion 22 of inspection apparatus 2 in the vertical direction during use.

Substrate main body 31 includes a plurality of through-holes 313 (see FIG. 4) that penetrate substrate main body 31 in the vertical direction.

A first end portion (in other words, upper end portion) of through-hole 313 is open to first surface 311 of substrate main body 31. A second end portion (in other words, lower end portion) of through-hole 313 is open to second surface 312 of substrate main body 31.

In the present embodiment, the central axis of through-hole 313 is parallel to the vertical direction. Furthermore, the cross-sectional shape of through-hole 313 is circular or oval.

Note that the cross-sectional shape of through-hole 313 is not limited to a circular or oval shape. The cross-sectional shape of through-hole 313 refers to the shape of through-hole 313 as viewed from above when substrate main body 31 is cut along a plane parallel to first surface 311 and second surface 312.

In the present embodiment, a plurality of columns (in the present embodiment, four columns) of through-holes 313 are provided in the left-right direction. One column includes a plurality of (in the present embodiment, 22) through-holes 313 arranged in the front-rear direction.

In the present embodiment, the positions of through-holes 313 in adjacent columns in the left-right direction are aligned with each other in the front-rear direction. However, the positions of through-holes 313 in adjacent columns in the left-right direction may be offset from each other in the front-rear direction.

That is, through-holes 313 may be arranged in a so-called staggered pattern. By arranging the through-holes in a staggered pattern, the spacing between terminal portions 35 of land portion 32 to be described later in the front-rear direction can be reduced. As a result, a large number of land portions 32 can be provided on substrate 3.

Furthermore, substrate main body 31 includes first metal layer 314 (see FIG. 4) around through-holes 313 on first surface 311. First metal layer 314 is made of copper. The shape of first metal layer 314 in plan view is the same as the shape of sensing portion 34 to be described later in plan view.

Furthermore, substrate main body 31 includes second metal layer 315 (see FIG. 4) around through-holes 313 on second surface 312. Second metal layer 315 is made of copper. The shape of second metal layer 315 in plan view is the same as the shape of terminal portion 35 to be described later in plan view.

Moreover, substrate main body 31 includes third metal layer 316 (see FIG. 5) on first surface 311 in a portion where dummy pad portion 36 to be described later is formed. Third metal layer 316 is made of copper. The shape of third metal layer 316 in plan view is the same as the shape of dummy pad portion 36 to be described later in plan view.

### (Land Portion)

Each of the plurality of land portions 32 is made of metal and is supported by substrate main body 31. Hereinafter, the configuration of one land portion 32 will be described. Land portion 32 is a portion that is electrically connected to inspection apparatus 2 during use (in other words, during inspection). In other words, the plurality of land portions 32 are lands used for inspection. Note that, in the present embodiment, the plurality of land portions 32 have the same configuration.

As illustrated in FIG. 4, land portion 32 includes land base portion 33, sensing portion 34, and terminal portion 35.

Land base portion 33 includes base main body 331, resin portion 332, first cover layer 333, and second cover layer 334. Land base portion 33 connects sensing portion 34 and terminal portion 35 to be described later. Such land base portion 33 corresponds to an example of a connecting portion.

Base main body 331 includes first base layer 331a, second base layer 331b, and connecting portion 331c.

First base layer 331a is provided on first surface 311 of substrate main body 31. Specifically, first base layer 331a is provided on the upper surface of first metal layer 314. First base layer 331a is made of copper. The shape of first base layer 331a in plan view is the same as the shape of first metal layer 314 in plan view.

Second base layer 331b is provided on second surface 312 of substrate main body 31. Specifically, second base layer 331b is provided on the lower surface of second metal layer 315. Second base layer 331b is made of copper. The shape of second base layer 331b in plan view is the same as the shape of second metal layer 315 in plan view.

Connecting portion 331c has a cylindrical shape and is disposed inside through-hole 313. Connecting portion 331c connects first base layer 331a and second base layer 331b. Connecting portion 331c is made of copper.

Base main body 331 having the above-described configuration is formed on substrate main body 31 by a plating process.

Resin portion 332 is shaft-shaped and passes through base main body 331. The central axis of resin portion 332 coincides with the central axis of through-hole 313. Note that resin portion 332 may be omitted. In this case, connecting portion 331c may have a columnar shape.

First cover layer 333 is provided on first surface 311 of substrate main body 31. Specifically, first cover layer 333 is provided on the upper surface of first base layer 331a. First cover layer 333 is made of copper.

The shape of first cover layer 333 in plan view is the same as the shapes of first metal layer 314 and first base layer 331a in plan view. Such first cover layer 333 is formed on the upper surface of first base layer 331a by a plating process.

Second cover layer 334 is provided on second surface 312 of substrate main body 31. Specifically, second cover layer 334 is provided on the lower surface of second base layer 331b. Second cover layer 334 is made of copper.

The shape of second cover layer 334 in plan view is the same as the shapes of second metal layer 315 and second base layer 331b in plan view. Such second cover layer 334 is formed on the lower surface of second base layer 331b by a plating process.

### (Sensing portion)

Sensing portion 34 is provided on first surface 311 of substrate main body 31. Specifically, sensing portion 34 is provided on the upper end surface of land base portion 33. More specifically, sensing portion 34 is provided on the upper surface of first cover layer 333 of land base portion 33. The shape of sensing portion 34 in plan view is the same as the shape of first cover layer 333 in plan view.

A region in which sensing portion 34 is provided on first surface 311 of substrate main body 31 is referred to as first region R₁. First region R₁ is a region surrounded by two-dot chain line α₁ in FIG. 2. First region R₁ is a rectangular region in plan view as illustrated in FIG. 2.

First region R₁ includes a portion where sensing portion 34 is provided and a portion where sensing portion 34 is not provided. The proportion of the area of the portion where sensing portion 34 is provided in the area of first region R₁ is defined as an area occupancy rate of sensing portion 34.

In the present embodiment, the area occupancy rate of sensing portion 34 is 17% or more. The area occupancy rate of sensing portion 34 is preferably 30% or more and 88% or less. The reason for adopting such a range of the area occupancy rate will be described later.

Sensing portion 34 is a portion that comes into contact with an object (in the present embodiment, target cells) to be measured for impedance during use (in other words, during inspection).

Sensing portion 34 is made of, for example, silver. Sensing portion 34 is constituted by a plating layer formed by an electroless plating method, on the upper surface of first cover layer 333 of land base portion 33.

Sensing portion 34 includes a silver chloride layer formed by a silver chloride treatment on the surface (in other words, upper surface). Such a silver chloride treatment can increase the surface area of sensing portion 34. As a result, the impedance of sensing portion 34 is reduced. The sensing portion may be made of gold. In this case, the sensing portion includes a platinum black layer formed by a platinum black treatment on the surface (in other words, upper surface).

Such sensing portion 34 has a desired thickness dimension. When the thickness dimension of sensing portion 34 is small, there is a possibility that copper constituting land base portion 33 and silver constituting sensing portion 34 may undergo a diffusion reaction when substrate 3 is exposed to a high-temperature environment for a long time. When such a diffusion reaction occurs, copper may precipitate on the surface of sensing portion 34, which may result in an increase in the impedance of sensing portion 34.

On the other hand, when the thickness dimension of sensing portion 34 is increased in order to suppress the above-described diffusion reaction, there is a possibility that the particles of silver constituting sensing portion 34 may increase in size. When the particles of silver constituting sensing portion 34 increase in size, the surface area of sensing portion 34 cannot be increased even with a silver chloride treatment applied to the surface of sensing portion 34. As a result, the impedance of sensing portion 34 may increase.

From this perspective, the thickness dimension of sensing portion 34 is preferably 0.1 µm or more and 0.8 µm or less. Further, the thickness dimension of sensing portion 34 is more preferably 0.1 µm or more and 0.6 µm or less. However, the thickness dimension of the sensing portion may be appropriately determined according to the specifications of substrate 3.

### (Terminal Portion)

Terminal portion 35 is provided at the lower end portion of base main body 331 of land base portion 33. Terminal portion 35 comes into contact with apparatus-side terminal portions 221 and 222 (see FIG. 4) of inspection apparatus 2 that measure the impedance of the object (in the present embodiment, target cells) during use (in other words, during inspection).

Terminal portion 35 is provided on second surface 312 of substrate main body 31. Specifically, terminal portion 35 is provided on the lower end surface of land base portion 33. More specifically, terminal portion 35 is provided on the lower surface of second cover layer 334 of land base portion 33.

The shape of terminal portion 35 in plan view is the same as the shape of second cover layer 334 in plan view. Specifically, the outer shape of terminal portion 35 is a shape along the inner peripheral surface of through-hole 313 (in the present embodiment, circular or oval shape).

Terminal portion 35 is made of, for example, silver. Terminal portion 35 is formed of a plating layer formed by an electroless plating method on the lower surface of second cover layer 334 of land base portion 33.

In the present embodiment, terminal portion 35 does not include a silver chloride layer on the surface (in other words, lower surface). However, terminal portion 35 may include a silver chloride layer formed by a silver chloride treatment on the surface (in other words, lower surface). Note that the terminal portion may be made of gold. In this case, the terminal portion includes a platinum black layer formed by a platinum black treatment on the surface (in other words, lower surface).

Such terminal portion 35 has a desired thickness dimension. When the thickness dimension of terminal portion 35 is small, there is a possibility that copper constituting land base portion 33 and silver constituting terminal portion 35 may undergo a diffusion reaction when exposed to a high-temperature environment for a long time. When such a diffusion reaction occurs, copper may precipitate on the surface of terminal portion 35, and the impedance of terminal portion 35 may increase.

On the other hand, when the thickness dimension of terminal portion 35 is increased in order to suppress the above-described diffusion reaction, there is a possibility that the particles of silver constituting terminal portion 35 may increase in size. When the particles of silver constituting terminal portion 35 increase in size, the surface area of terminal portion 35 cannot be increased. As a result, the impedance of terminal portion 35 may increase.

From this perspective, the thickness dimension of terminal portion 35 is preferably 0.1 µm or more and 0.8 µm or less. Further, the thickness dimension of terminal portion 35 is more preferably 0.1 µm or more and 0.6 µm or less.

### (Dummy Pad Portion)

Next, a configuration of dummy pad portion 36 will be described with reference to FIGS. 2 and 5. Dummy pad portion 36 has a rectangular frame shape in plan view illustrated in FIG. 2. Such dummy pad portion 36 is a portion that is not electrically connected to inspection apparatus 2 during use (in other words, during inspection). In other words, dummy pad portion 36 is a land that is not used during inspection.

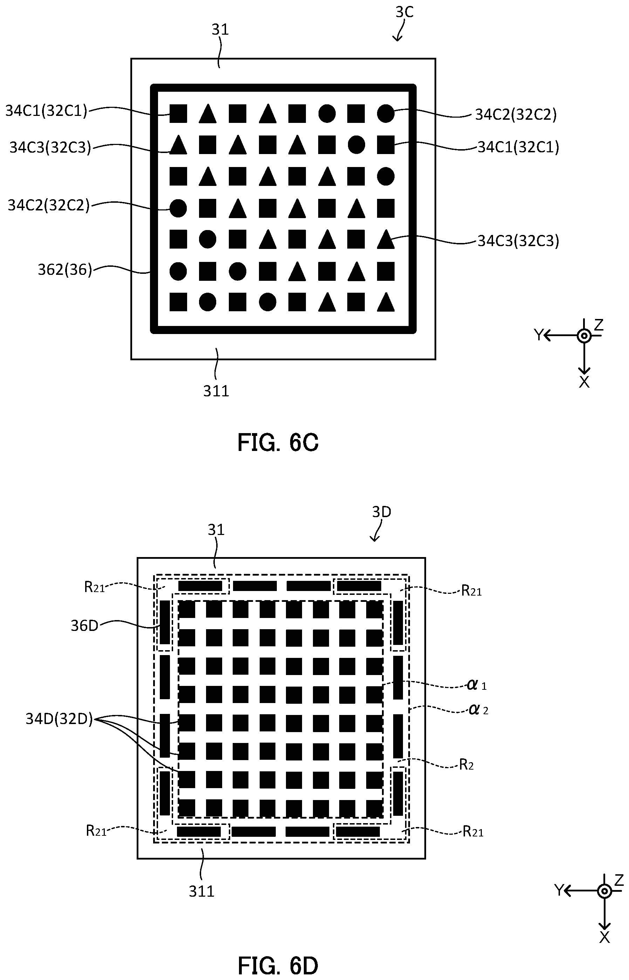

Dummy pad portion 36 is continuous over the entire circumference. In other words, dummy pad portion 36 has a continuous rectangular frame shape. Note that the dummy pad portion may include a discontinuous portion (see FIG. 6D) in a part thereof. In other words, the dummy pad portion may have a discontinuous rectangular frame shape.

Dummy pad portion 36 is provided on first surface 311 (in other words, upper surface) of substrate main body 31. Specifically, dummy pad portion 36 is provided in second region R₂ on first surface 311.

Second region R₂ is a rectangular frame-shaped region that surrounds first region R₁ (see FIG. 2) in which sensing portion 34 is provided on first surface 311. Second region R₂ is a region surrounded by two-dot chain line α₁ and two-dot chain line α₂ in FIG. 2.

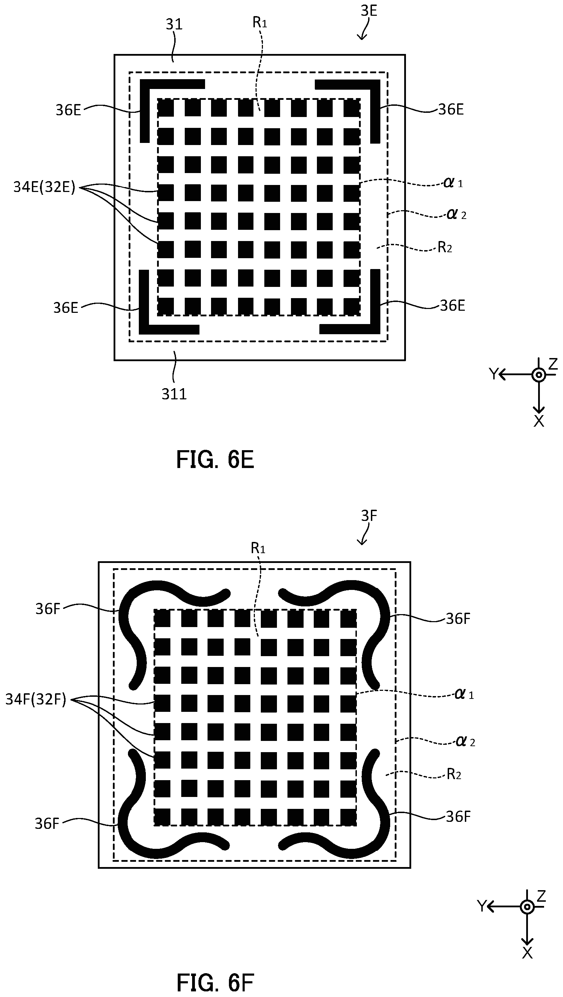

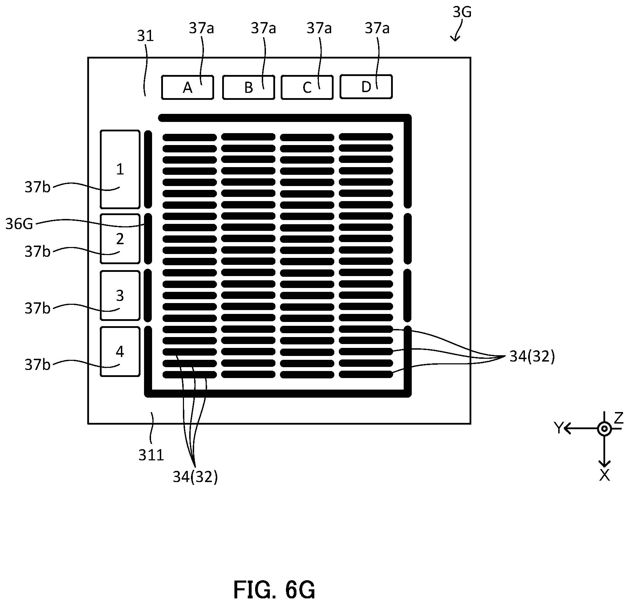

In the present embodiment, dummy pad portion 36 is provided to extend along the entire circumference of second region R₂ of first surface 311. Note that, as illustrated in FIGS. 6D, 6E, 6F, and 6G, the dummy pad portion may be provided in a predetermined region including at least four corners in second region R₂ of first surface 311. Note that the predetermined region including at least four corners may be, for example, four regions R₂₁ surrounded by a two-dot chain line in FIG. 6D.

As illustrated in FIG. 5, dummy pad portion 36 is provided only on first surface 311 of substrate main body 31. In other words, as illustrated in FIG. 5, dummy pad portion 36 is not provided on second surface 312 of substrate main body 31.

Dummy pad portion 36 has a predetermined width dimension W₁ (see FIG. 2). In the present embodiment, width dimension W₁ of dummy pad portion 36 is 0.10 mm or more. Further, width dimension W₁ of dummy pad portion 36 is preferably 0.25 mm or more. The reason for adopting such width dimension W₁ will be described later.

In the present embodiment, width dimension W₁ of dummy pad portion 36 is constant over the entire circumference of dummy pad portion 36. However, width dimension W₁ of dummy pad portion 36 may be partially different.

Further, the distance between sensing portion 34 and dummy pad portion 36 is distance Di (see FIG. 2). Distance Di is a distance between dummy pad portion 36 and sensing portion 34 of land portion 32 located at the outermost edge of first region R₁ among the plurality of land portions 32, as illustrated in FIG. 2.

In the present embodiment, distance Di is 0.025 mm or more, preferably 0.1 mm or more, and more preferably 0.25 mm or more and 1.00 mm or less. The reason for adopting such distance Di will be described later.

Specifically, as illustrated in FIG. 5, dummy pad portion 36 includes base portion 361 and plating layer 362. Base portion 361 has a rectangular frame shape and is provided in second region R₂ of first surface 311 of substrate main body 31. Specifically, base portion 361 is provided on the upper surface of third metal layer 316 provided on first surface 311 of substrate main body 31.

Base portion 361 includes first base layer 361a and first cover layer 361b.

First base layer 361a is provided on first surface 311 of substrate main body 31. Specifically, first base layer 361a is provided on the upper surface of third metal layer 316. First base layer 361a is formed of copper.

The shape of first base layer 361a in plan view is the same as the shape of third metal layer 316 in plan view. First base layer 361a is formed of copper. Such first base layer 361a is formed on substrate main body 31 by a plating process.

First cover layer 361b is provided on first surface 311 of substrate main body 31. Specifically, first cover layer 361b is provided on the upper surface of first base layer 361a. First cover layer 361b is formed of copper.

The shape of first cover layer 361b in plan view is the same as the shape of third metal layer 316 and first base layer 361a in plan view. Such first cover layer 361b is formed on the upper surface of first base layer 361a by a plating process.

Plating layer 362 is provided on the surface (in other words, upper surface) of base portion 361 (specifically, first cover layer 361b). Plating layer 362 is formed of silver. Plating layer 362 is formed of a plating layer formed on the surface (in other words, upper surface) of base portion 361 by an electroless plating method.

Plating layer 362 includes a silver chloride layer formed on the surface (in other words, upper surface) by a silver chloride treatment. Note that the plating layer may be made of gold. In this case, the plating layer includes a platinum black layer formed on the surface (in other words, upper surface) by a platinum black treatment. Alternatively, the silver chloride layer may be omitted.

Dummy pad portion 36 having the above-described configuration contributes to reducing variation in the thickness dimension of sensing portion 34 between land portions 32 when sensing portions 34 of substrate 3 are formed. The effects of dummy pad portion 36 will be described later.

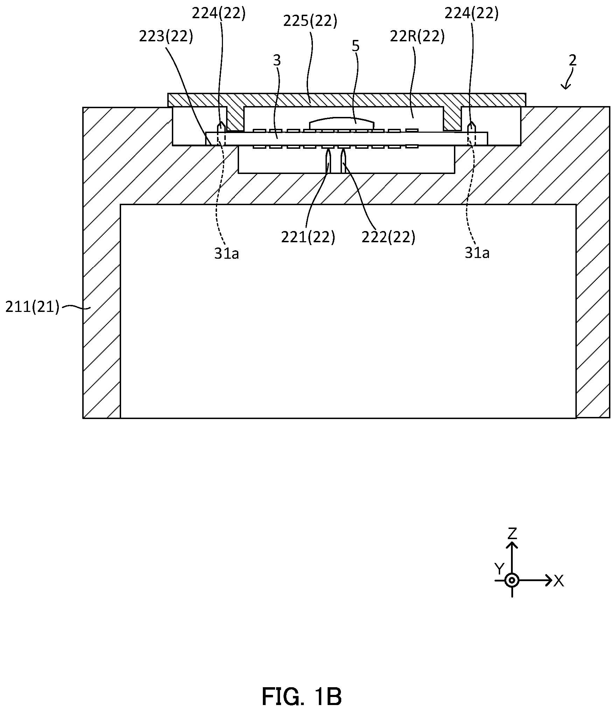

Substrate 3 having the above-described configuration is placed on mounting portion 223 of substrate support portion 22 in inspection apparatus 2 (see FIG. 1A) during use (in other words, during inspection). In this state, as illustrated in FIGS. 1B and 4, apparatus-side terminal portions 221 and 222 of inspection apparatus 2 are in contact with terminal portions 35 of land portions 32 in substrate 3, respectively.

That is, apparatus-side terminal portions 221 and 222 of inspection apparatus 2 and land portions 32 of substrate 3 are electrically connected to each other. In the present embodiment, land portion 32 in conduction with apparatus-side terminal portion 221 of inspection apparatus 2 and land portion 32 in conduction with apparatus-side terminal portion 222 of inspection apparatus 2 are disposed adjacent to each other.

Hereinafter, for convenience of description, land portion 32 in conduction with apparatus-side terminal portion 221 of inspection apparatus 2 is referred to as land portion 32a (see FIG. 4). On the other hand, land portion 32 in conduction with apparatus-side terminal portion 222 of inspection apparatus 2 is referred to as land portion 32b (see FIG. 4).

Then, the user places target cells 5 (see FIGS. 1B and 4) on first surface 311 (that is, upper surface) of substrate 3. Specifically, the user places target cells 5 above land portions 32a and 32b so as to straddle land portions 32a and 32b.

Since target cells 5 have conductivity, land portions 32a and 32b are electrically connected to each other. As a result, a current flows between apparatus-side terminal portion 221 and apparatus-side terminal portion 222 of inspection apparatus 2 via land portion 32a, target cell 5, and land portion 32b.

Inspection apparatus 2 measures the impedance of target cells 5 based on the current flowing between apparatus-side terminal portion 221 and apparatus-side terminal portion 222. Then, inspection apparatus 2 identifies the type of target cells 5 based on the measured impedance of target cells 5. Specifically, it is identified whether target cells 5 are cancer cells.

When the inspection is completed, substrate 3 is discarded after one use. That is, substrate 3 is a disposable substrate. In a case where a large number of unused land portions 32 remain, substrate 3 may be used a plurality of times.

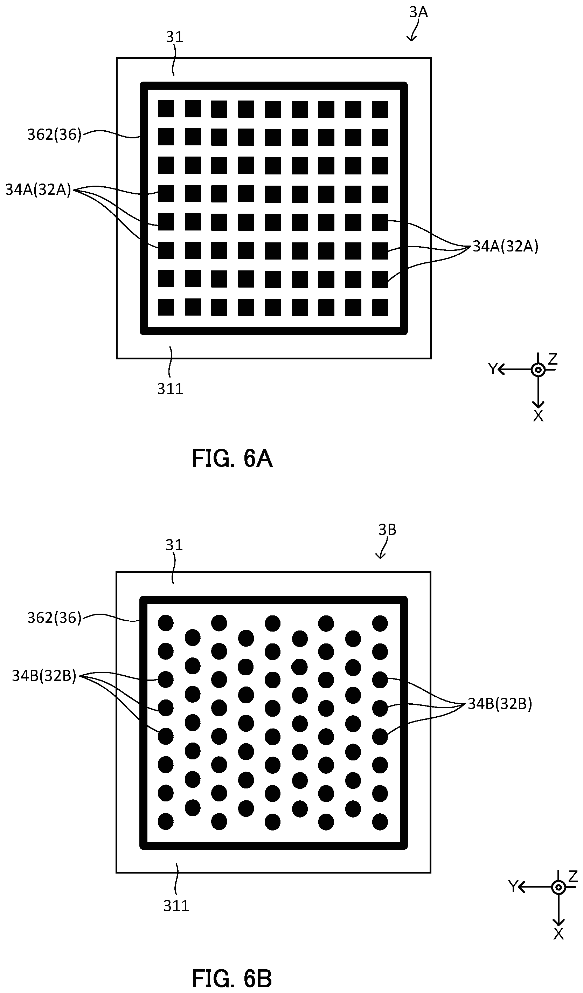

The use of substrate 3 is not limited to the above-described use. Substrate 3 can be applied to various apparatuses that measure the characteristics of an object. Further, the configuration of the substrate is not limited to that of the above-described substrate 3. Hereinafter, variations of the substrate will be described. FIGS. 6A to 6G are diagrams illustrating Variations 1 to 7 of the substrate.

### (Variation 1)

FIG. 6A is a plan view of substrate 3A according to Variation 1 of the substrate. In substrate 3A, the configuration of land portion 32A is different from that of land portion 32 in the above-described embodiment.

Specifically, in the case of land portion 32A, the shape of sensing portion 34A in plan view is quadrilateral (square or rectangular). For the land base portion of land portion 32A, the description of land base portion 33 in FIG. 4 may be referred to as appropriate. The other configurations of substrate 3A are the same as the configurations of substrate 3 in the embodiment.

### (Variation 2)

FIG. 6B is a plan view of substrate 3B according to Variation 2 of the substrate. In substrate 3B, the configuration of land portion 32B is different from that of land portion 32 in the above-described embodiment.

Specifically, in the case of land portion 32B, the shape of sensing portion 34B in plan view is circular. For the land base portion of land portion 32B, the description of land base portion 33 in FIG. 4 may be referred to as appropriate. The other configurations of substrate 3B are the same as the configurations of substrate 3 in the embodiment.

### (Variation 3)

FIG. 6C is a plan view of substrate 3C according to Variation 3 of the substrate. In substrate 3C, the configurations of land portions 32C1 to 32C3 are different from the configuration of land portion 32 in the above-described embodiment. Specifically, substrate 3C includes three types of land portions 32C1 to 32C3.

In the case of land portion 32C1, the shape of sensing portion 34C1 in plan view is quadrilateral (square or rectangular). For the land base portion of land portion 32C1, the description of land base portion 33 in FIG. 4 may be referred to as appropriate.

In the case of land portion 32C2, the shape of sensing portion 34C2 in plan view is circular. For the land base portion of land portion 32C2, the description of land base portion 33 in FIG. 4 may be referred to as appropriate.

In the case of land portion 32C3, the shape of sensing portion 34C3 in plan view is a triangle. For the land base portion of land portion 32C3, the description of land base portion 33 in FIG. 4 may be referred to as appropriate.

The arrangement order of land portions 32C1 to 32C3 may be regular or irregular. As described above, the substrate may include a plurality of types of land portions. The other configurations of substrate 3C are the same as the configurations of substrate 3 in the embodiment.

### (Variation 4)

FIG. 6D is a plan view of substrate 3D according to Variation 4 of the substrate. In substrate 3D, the configuration of land portion 32D and the configuration of dummy pad portion 36D are respectively different from the configuration of land portion 32 and the configuration of dummy pad portion 36 in the above-described embodiment.

Specifically, in the case of land portion 32D, the shape of sensing portion 34D in plan view is quadrilateral (square or rectangular). For the land base portion of land portion 32D, the description of land base portion 33 in FIG. 4 may be referred to as appropriate.

In addition, dummy pad portion 36D has a discontinuous rectangular frame shape. In other words, dummy pad portion 36D has discontinuous portions. In the present embodiment, dummy pad portion 36D is provided in a predetermined region including at least four corners in second region R₂ of first surface 311 in substrate main body 31.

Note that the predetermined region including at least four corners may be, for example, four regions R₂₁ surrounded by a two-dot chain line in FIG. 6D. As described above, the shape of dummy pad portion 36D may be any of various shapes. The other configurations of substrate 3D are the same as the configurations of substrate 3 in the embodiment.

### (Variation 5)

FIG. 6E is a plan view of substrate 3E according to Variation 5 of the substrate. In substrate 3E, the configuration of land portion 32E and the configuration of dummy pad portion 36E are respectively different from the configuration of land portion 32 and the configuration of dummy pad portion 36 in the above-described embodiment.

Specifically, in the case of land portion 32E, the shape of sensing portion 34E in plan view is quadrilateral (square or rectangular). For the land base portion of land portion 32E, the description of land base portion 33 in FIG. 4 may be referred to as appropriate.

In addition, dummy pad portion 36E is provided in parts of second region R₂ of first surface 311 in substrate main body 31. Specifically, dummy pad portion 36E is provided only in a predetermined region including the four corners of second region R₂.

Each of dummy pad portions 36E has an angular line shape bent at a right angle along each of the four corners of second region R₂. Dummy pad portion 36E in the present variation may be regarded as a discontinuous rectangular frame-shaped dummy pad portion. The other configurations of substrate 3E are the same as the configurations of substrate 3 in the embodiment.

### (Variation 6)

FIG. 6F is a plan view of substrate 3F according to Variation 6 of the substrate. In substrate 3F, the configuration of land portion 32F and the configuration of dummy pad portion 36F are respectively different from the configuration of land portion 32 and the configuration of dummy pad portion 36 in the above-described embodiment.

Specifically, in the case of land portion 32F, the shape of sensing portion 34F in plan view is quadrilateral (square or rectangular). For the land base portion of land portion 32F, the description of land base portion 33 in FIG. 4 may be referred to as appropriate.

In addition, dummy pad portion 36F is provided in parts of second region R₂ of first surface 311 in substrate main body 31. Specifically, dummy pad portion 36F is provided only in a predetermined region including the four corners of second region R₂.

Each of dummy pad portions 36F has a curved shape bent along the four corners of second region R₂. As described above, the shape of dummy pad portion 36F may be any of various shapes. The other configurations of substrate 3F are the same as the configurations of substrate 3 in the embodiment.

### (Variation 7)

FIG. 6G is a plan view of substrate 3G according to Variation 7 of the substrate. In substrate 3G, the configuration of dummy pad portion 36G is different from that of dummy pad portion 36 in the above-described embodiment.

In addition, substrate 3G includes horizontal symbols 37a and vertical symbols 37b around dummy pad portion 36. Horizontal symbols 37a and vertical symbols 37b are symbols for a user to specify a region on substrate 3 to be used during inspection.

In other words, horizontal symbols 37a and vertical symbols 37b are symbols for specifying sensing portion 34 to be used during inspection. Note that, in this case, the horizontal direction coincides with the left-right direction of substrate 3G. Further, the vertical direction coincides with the front-rear direction of substrate 3G.

For example, when using sensing portion 34 included in region A-1, the user inputs a symbol "A" of horizontal symbol 37a and a symbol "1" of vertical symbol 37b into inspection apparatus 2. Inspection apparatus 2 measures the impedance of target cells using sensing portion 34 included in region A-1 specified by the user.

In the case of the present variation, portions of dummy pad portion 36G corresponding to the right side and the left side are divided corresponding to vertical symbols 37b. By looking at the division positions of dummy pad portion 36G, the user can intuitively recognize the vertical regions in substrate 3G.

Dummy pad portion 36G in this variation as described above may be regarded as a discontinuous rectangular frame-shaped dummy pad portion. The other configurations of substrate 3G are the same as the configurations of substrate 3 in the embodiment.

### (Examples)

Hereinafter, examples of substrate 3 according to the present invention and tests performed to confirm the effects of substrate 3 according to the present embodiment will be described. The present invention is not limited to these examples and tests.

The inventors of the present invention performed a test to confirm the functions and effects obtained by providing dummy pad portion 36 on substrate 3.

### (1) Method for Manufacturing Test Piece

First, the inventors prepared a plurality of test pieces. As a base material of the test pieces, a copper-clad laminate for a so-called printed circuit board was used. Specifically, L-6705C1 manufactured by NIKKAN INDUSTRIES CO., LTD. was used as the base material (not shown). The thickness dimension of the base material was 1.6 mm. In addition, the thickness dimension of a copper foil provided on the surface of the base material was 18 µm.

Such a base material was cut to obtain a rectangular plate-shaped workpiece-size substrate (not shown). The horizontal dimension of the work-size substrate was 40 cm. In addition, the vertical dimension of the work-size substrate was 25 cm. On the surface (specifically, first surface) of the work-size substrate obtained in this manner, patterns corresponding to land base portion 33 of land portion 32 and base portion 361 of dummy pad portion 36 in substrate 3 were formed.

Note that this test is for examining an impact on variation in the thickness dimension of sensing portions 34 caused by providing dummy pad portion 36 on substrate 3. Thus, the test piece is a test piece in which configurations corresponding to through-hole 313 and terminal portion 35 of substrate main body 31 in the above-described embodiment are omitted.

### (1.1) Pattern Formation

Hereinafter, a method for forming land base portion 33 of land portion 32 and base portion 361 of dummy pad portion 36 on the work-size substrate will be described. First, a first intermediate work-size substrate (not shown) was prepared by performing a soft etching process as pre-treatment polishing on the work-size substrate. Then, a dry film was adhered to the surface of the first intermediate work-size substrate to prepare a second intermediate work-size substrate (not shown).

Next, with a circuit print film placed on the surface of the second intermediate work-size substrate, exposure was performed by irradiation with a UV lamp to cure only pattern portions of the dry film, thereby preparing a third intermediate work-size substrate (not shown). Note that portions of the dry film that were not cured were removed with a developer.

Next, an etching process was performed on the third intermediate work-size substrate to remove unnecessary copper from the third intermediate work-size substrate, thereby preparing a fourth intermediate work-size substrate (not shown). A copper pattern was formed on the surface of the fourth intermediate work-size substrate.

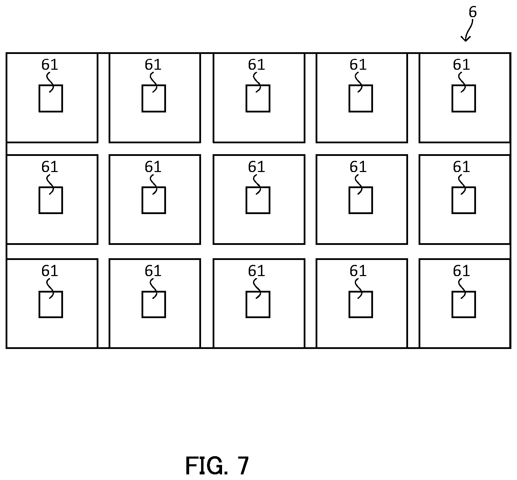

Next, the fourth intermediate work-size substrate was immersed in a sodium hydroxide solution to strip the dry film from the fourth intermediate work-size substrate and expose the copper pattern, and then copper plating as an underlayer was deposited to a thickness of 15 µm, thereby obtaining fifth intermediate work-size substrate 6 (see FIG. 7). This copper plating corresponds to first cover layer 333 in land portion 32 described above. This copper plating also corresponds to first cover layer 361b in dummy pad portion 36 described above.

Fifth intermediate work-size substrate 6 has fifteen types of pattern portions 61 (see FIG. 7) on the surface thereof. Pattern portions 61 may be regarded as patterns corresponding to land base portions 33 (see FIGS. 4 and 5) of a plurality of land portions 32 and base portion 361 of dummy pad portion 36 in substrate 3. The interval in the horizontal direction and the interval in the vertical direction between pattern portions 61 were each 5 cm or more.

### (1.2) Plating Process

By performing a plating process on pattern portions 61 formed on the surface of fifth intermediate work-size substrate 6 obtained as described above, a plating layer corresponding to sensing portions 34 (see FIG. 4) of the plurality of land portions 32 and plating layer 362 of dummy pad portion 36 was formed on the surfaces (specifically, upper surfaces) of pattern portions 61.

In the case of the present example, a silver plating solution was used as the plating solution. As the silver plating solution, AG-100B manufactured by MacDermid Performance Solutions Japan K.K. was used.

The target final thickness dimension of the plating layer was 0.5 µm. Note that the target final thickness dimension of the plating layer is a target final thickness dimension of sensing portion 34 of land portion 32 to be formed by the plating process. The thickness dimension of sensing portion 34 actually formed may deviate from 0.5 µm depending on various conditions of the plating process.

A pre-plating process was performed on fifth intermediate work-size substrate 6. The temperature of the silver plating solution in the pre-plating process was 52°C. The duration of the pre-plating process was 30 seconds.

Next, a main plating process was performed on fifth intermediate work-size substrate 6 after the pre-plating process. The temperature of the silver plating solution in the main plating process was 52°C. The duration of the main plating process was 2 minutes and 24 seconds.

An operator continued to agitate fifth intermediate work-size substrate 6 during the main plating process. After the main plating process, a cleaning process (city water cleaning and pure water cleaning), a drying process, and a laminating process were performed on fifth intermediate work-size substrate 6 to obtain a final work-size substrate (not shown). In the laminating process, a protective film was laminated over the entire surface of fifth intermediate work-size substrate 6.

### (1.3) Cutting Process

The final work-size substrate obtained as described above was cut into test pieces of a predetermined size. The horizontal dimension of the test piece was 70 mm. In addition, the vertical dimension of the test piece was 70 mm. After the cutting process, the protective film was peeled from the surfaces of the test pieces.

### (2) Regarding tests

Conventionally, a substrate from which dummy pad portion 36, through-hole 313, terminal portion 35, and the like are omitted from substrate 3 illustrated in FIG. 2 (hereinafter referred to as "substrate of a conventional structure") is known. Such a substrate of a conventional structure differs from the configuration of substrate 3 described above in that dummy pad portion 36, through-hole 313, and terminal portion 35 are not provided, but is manufactured by substantially the same manufacturing method as the manufacturing method described above.

The inventors found that, in the case of the substrate of the conventional structure, variation in the thickness dimension of sensing portions 34 (see FIG. 2) is large. Specifically, the inventors found that, in the case of the substrate of the conventional structure, the thickness dimension of sensing portions 34 at corner portions becomes larger than the thickness dimension of sensing portions 34 at a center portion.

Note that sensing portions 34 at the center portion are four sensing portions 34 provided in a region surrounded by a two-dot chain line R₃ in FIG. 2. In addition, sensing portions 34 at the corner portions are four sensing portions 34 at the four corners among sensing portions 34 in FIG. 2.

The inventors considered that one of the causes of the variation in the thickness dimension of sensing portions 34 is that, in the plating process described above, an ion concentration (e.g., silver ion concentration) of a plating solution (e.g., silver plating solution) forming sensing portions 34 at the corner portions becomes higher than the ion concentration (e.g., silver ion concentration) of the plating solution (e.g., silver plating solution) forming sensing portions 34 at the center portion.

Therefore, in order to lower the ion concentration of the plating solution forming sensing portions 34 at the corner portions in the plating process described above, the inventors conceived an idea regarding substrate 3 according to the present embodiment in which dummy pad portion 36 is provided around sensing portions 34 at the corner portions.

Then, the inventors conducted tests for confirming the functions and effects exhibited by substrate 3 according to the present embodiment.

### (First Test)

First, the inventors conducted a test (hereinafter referred to as "first test") for confirming the impact of the presence or absence of the dummy pad portion on the variation in the thickness dimension of sensing portions 34. The first test is also a test for confirming the impact of the area occupancy rate of sensing portions 34 on the variation in the thickness dimension of sensing portions 34. For the first test, the inventors prepared test pieces S1 to S14 by the manufacturing method described above.

Test pieces S1 to S4 are substrates that do not include a dummy pad portion. Such test pieces S1 to S4 correspond to reference examples. On the other hand, test pieces S5 to S14 are substrates that include a dummy pad portion. Such test pieces S5 to S14 correspond to examples. Note that the configurations of test pieces S1 to S14 are sometimes described using the configuration of substrate 3 illustrated in FIG. 2.

Conditions for test pieces S1 to S14 are shown in Table 1 below.

**[Table 1]**

| Test piece No. | Dummy pad portion | Area occupancy rate | Width dimension W₁ | Distance D₁ | Ratio R |
|---|---|---|---|---|---|
| 1 | Absent | 23% | 0.75 mm | 0.50 mm | 0.95 |
| 2 | Absent | 46% | 0.75 mm | 0.50 mm | 0.79 |
| 3 | Absent | 70% | 0.75 mm | 0.50 mm | 0.73 |
| 4 | Absent | 100% | 0.75 mm | 0.50 mm | 0.69 |
| 5 | Present | 23% | 0.75 mm | 0.50 mm | 0.98 |
| 6 | Present | 31% | 0.75 mm | 0.50 mm | 0.97 |
| 7 | Present | 46% | 0.75 mm | 0.50 mm | 0.89 |
| 8 | Present | 50% | 0.75 mm | 0.50 mm | 0.86 |
| 9 | Present | 57% | 0.75 mm | 0.50 mm | 0.88 |
| 10 | Present | 65% | 0.75 mm | 0.50 mm | 0.81 |
| 11 | Present | 70% | 0.75 mm | 0.50 mm | 0.81 |
| 12 | Present | 75% | 0.75 mm | 0.50 mm | 0.78 |
| 13 | Present | 82% | 0.75 mm | 0.50 mm | 0.75 |
| 14 | Present | 100% | 0.75 mm | 0.50 mm | 0.74 |

The area occupancy rate in Table 1 above is a ratio of sensing portions 34 to first region R₁ (see FIG. 2) on first surface 311 (in other words, upper surface) of substrate main body 31.

Width dimension Wi in Table 1 above is the width dimension of dummy pad portion 36 (see FIG. 2). In addition, distance Di in Table 1 above is distance Di (see FIG. 2) between sensing portion 34 and dummy pad portion 36. As illustrated in FIG. 2, distance Di is a distance between dummy pad portion 36 and sensing portion 34 of land portion 32 located at the outermost edge of first region R₁ among the plurality of land portions 32.

In the first test, the inventors measured the thickness dimension of sensing portions 34 in test pieces S1 to S14 as described above. Then, the inventors determined, for test pieces S1 to S14, ratio R of thickness dimension T₁ of sensing portions 34 at the center portion to thickness dimension T₂ of sensing portions 34 at the corner portions. Ratio R was determined by the following Equation 1.

Note that sensing portions 34 at the center portion are four sensing portions 34 provided in the region surrounded by two-dot chain line R₃ in FIG. 2. Thickness dimension T₁ of sensing portions 34 at the center portion was an average value of the thickness dimensions of these four sensing portions 34.

In addition, sensing portions 34 at the corner portions are four sensing portions 34 at the four corners among sensing portions 34 in FIG. 2. Thickness dimension T₂ of sensing portions 34 at the corner portions was an average value of the thickness dimensions of these four sensing portions 34.

[1]$R = {T}_{1} / {T}_{2}$ ... (Equation 1)

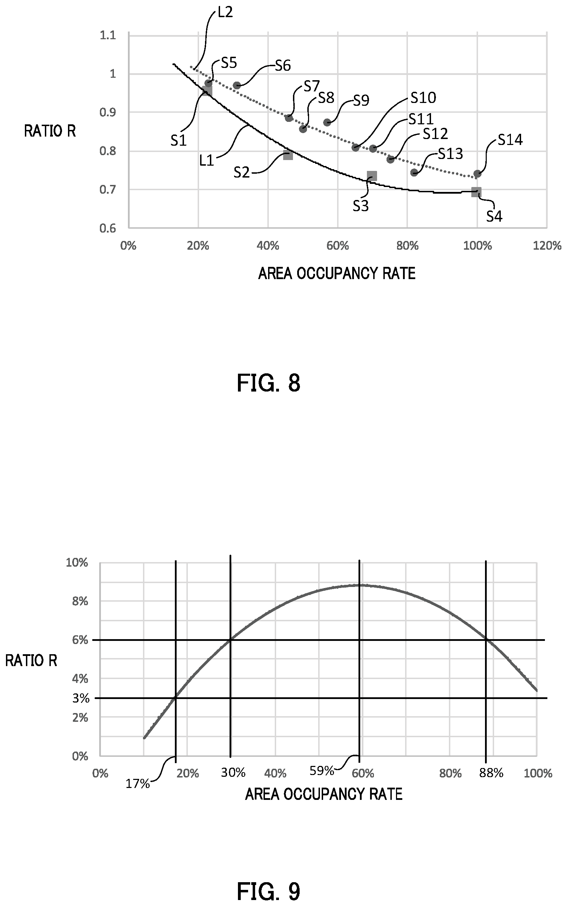

FIG. 8 is a line graph showing ratios R of test pieces S1 to S14 calculated based on the measurement results. In FIG. 8, the horizontal axis indicates the area occupancy rate. In FIG. 8, the vertical axis indicates ratio R. FIG. 8 shows ratio R for each of test pieces S1 to S14. Reference numerals S1 to S14 shown in FIG. 8 indicate ratios R of test pieces S1 to S14.

In FIG. 8, approximation line L1 shows an approximation line obtained by approximating ratios R of test pieces S1 to S4, which are the reference examples, with a quadratic curve. In addition, in FIG. 8, approximation line L2 shows an approximation line obtained by approximating ratios R of test pieces S5 to S14, which are the examples, with a quadratic curve.

As is clear from approximation lines L1 and L2 shown in FIG. 8, approximation line L2 showing ratios R of test pieces S5 to S14, which are the examples, is located overall on the upper side of approximation line L1 showing ratios R of test pieces S1 to S4, which are the reference examples.

This means that the difference between thickness dimension T₁ of sensing portions 34 at the center portion and thickness dimension T₂ of sensing portions 34 at the corner portions is smaller in test pieces S5 to S14 provided with dummy pad portion 36 than in test pieces S1 to S4 not provided with dummy pad portion 36.

In other words, the relationship between approximation line L1 and approximation line L2 shown in FIG. 8 means that the variation in the thickness dimension of sensing portions 34 regarding test pieces S5 to S14 is smaller than the variation in the thickness dimension of sensing portions 34 regarding test pieces S1 to S4.

From the above result, the inventors confirmed that the variation in the thickness dimension of sensing portions 34 is reduced by providing dummy pad portion 36.

In addition, as shown in FIG. 8, approximation line L2 is located overall on the upper side of approximation line L1. As a result, the inventors confirmed that, regardless of the area occupancy rate, the variation in the thickness dimension of sensing portions 34 is reduced by the provision of the dummy pad portion.

FIG. 9 is a diagram showing a difference between approximation line L2 and approximation line L1 in FIG. 8. From FIG. 9, it was found that when the area occupancy rate is 17% or more, the difference between approximation line L2 and approximation line L1 is 3% or more. Thus, the area occupancy rate of sensing portions 34 is preferably 17% or more.

Further, from FIG. 9, it was found that when the area occupancy rate is 30% or more and 88% or less, the difference between approximation line L2 and approximation line L1 is 6% or more. Thus, the area occupancy rate of sensing portions 34 is more preferably 30% or more and 88% or less. Note that the peak of the difference between approximation line L2 and approximation line L1 was 8.8%. The area occupancy rate of sensing portions 34 in this case was 59%.

### (Second Test)

Next, the inventors conducted a test (hereinafter referred to as "second test") for confirming the impact of distance Di (see FIG. 2) between the dummy pad portion and the sensing portion on the variation in the thickness dimension of sensing portions 34. For the second test, the inventors prepared test pieces S15 to S22 by a method similar to the manufacturing method described above.

Conditions for test pieces S15 to S22 are shown in Table 2 below.

**[Table 2]**

| Test piece No. | Dummy pad portion | Area occupancy rate | Width dimension W₁ | Distance Di | Ratio R |
|---|---|---|---|---|---|
| 15 | Present | 46% | 0.75 mm | 0.25 mm | 0.92 |
| 16 | Present | 46% | 0.75 mm | 0.50 mm | 0.89 |
| 17 | Present | 46% | 0.75 mm | 0.75 mm | 0.92 |
| 18 | Present | 46% | 0.75 mm | 1.00 mm | 0.85 |
| 19 | Present | 57% | 0.75 mm | 0.25 mm | 0.92 |
| 20 | Present | 57% | 0.75 mm | 0.50 mm | 0.88 |
| 21 | Present | 57% | 0.75 mm | 0.75 mm | 0.83 |
| 22 | Present | 57% | 0.75 mm | 1.00 mm | 0.82 |

Then, the thickness dimensions of sensing portions 34 in test pieces S15 to S22 were measured, and ratio R of thickness dimension T₁ of sensing portions 34 at the center portion to thickness dimension T₂ of sensing portions 34 at the corner portions was determined. Ratio R was determined by Equation 1 above, similar to the first test.

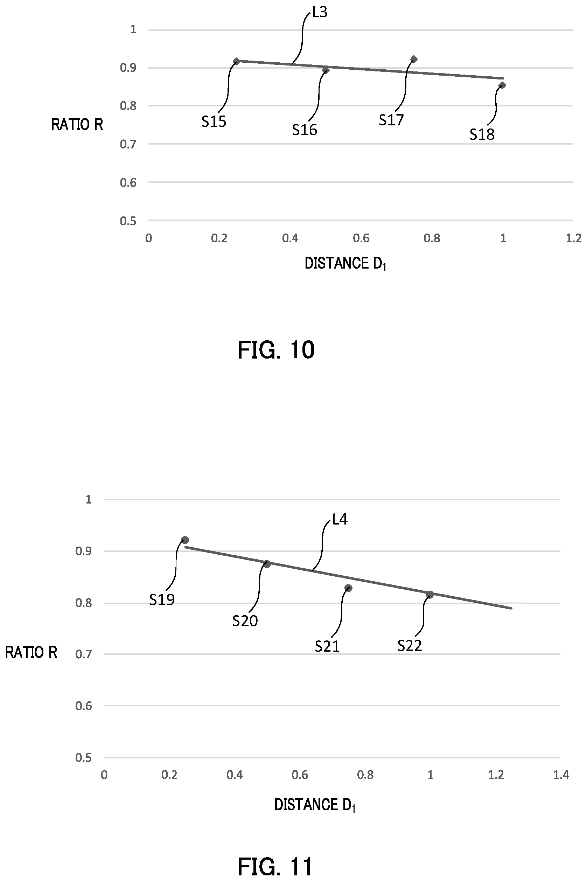

FIG. 10 is a diagram showing ratios R of test pieces S15 to S18 calculated based on the measurement results. In FIG. 10, the horizontal axis indicates distance D₁. In addition, in FIG. 10, the vertical axis indicates ratio R. FIG. 10 shows ratio R for each of test pieces S15 to S18. Reference numerals S15 to S18 shown in FIG. 10 indicate ratios R of test pieces S15 to S18. In FIG. 10, approximation line L3 shows an approximation line obtained by approximating ratios R of test pieces S15 to S18 with a straight line.

FIG. 11 is a diagram showing ratios R of test pieces S19 to S22 calculated based on the measurement results. In FIG. 11, the horizontal axis indicates distance D₁. In addition, in FIG. 11, the vertical axis indicates ratio R. FIG. 11 shows ratio R for each of test pieces S19 to S22. Reference numerals S19 to S22 shown in FIG. 11 indicate ratios R of test pieces S19 to S22. In FIG. 11, approximation line L4 shows an approximation line obtained by approximating ratios R of test pieces S19 to S22 with a straight line.

As shown in FIGS. 10 and 11, ratios R of test pieces S15 to S18 and ratios R of test pieces S19 to S22 are 0.8 or more in a range in which distance Di is 0.25 mm or more and 1.00 mm or less. In addition, as shown in FIGS. 10 and 11, ratios R of test pieces S15 to S18 and ratios R of test pieces S19 to S22 tend to decrease as distance D₁ increases.

In other words, the variation in the thickness dimension of sensing portions 34 tends to increase as distance Di increases. When ratio R is 0.8 or more, the variation in the thickness dimension of sensing portions 34 is sufficiently small. Thus, distance Di is preferably 0.25 mm or more and 1.00 mm or less. Although the lower limit value of distance Di has not been confirmed in this test, it is preferably as small as possible. As a realistic range of distance Di for maintaining insulation between sensing portion 34 and dummy pad portion 36, the lower limit value of distance Di may be 0.025 mm or more, and more practically 0.1 mm or more.

### (Third Test)

Next, the inventors conducted a test (hereinafter referred to as "third test") for examining the impact of width dimension W₁ (see FIG. 2) of the dummy pad portion on the variation in the thickness dimension of sensing portions 34. The inventors prepared test pieces S23 to S32 by a method similar to the manufacturing method described above.

Conditions for test pieces S23 to S32 are shown in Table 3 below.

**[Table 3]**

| Test piece No. | Dummy pad portion | Area occupancy rate | Width dimension W₁ | Distance Di | Ratio R |
|---|---|---|---|---|---|
| 23 | Present | 46% | 0.25 mm | 0.50 mm | 0.85 |
| 24 | Present | 46% | 0.50 mm | 0.50 mm | 0.95 |
| 25 | Present | 46% | 0.75 mm | 0.50 mm | 0.98 |
| 26 | Present | 46% | 1.00 mm | 0.50 mm | 0.90 |
| 27 | Present | 46% | 1.25 mm | 0.50 mm | 0.96 |
| 28 | Present | 57% | 0.25 mm | 0.50 mm | 0.87 |
| 29 | Present | 57% | 0.50 mm | 0.50 mm | 0.90 |
| 30 | Present | 57% | 0.75 mm | 0.50 mm | 0.88 |
| 31 | Present | 57% | 1.00 mm | 0.50 mm | 0.90 |
| 32 | Present | 57% | 1.25 mm | 0.50 mm | 0.92 |

Then, the thickness dimensions of sensing portions 34 in test pieces S23 to S32 were measured, and ratio R of thickness dimension T₁ of sensing portions 34 at the center portion to thickness dimension T₂ of sensing portions 34 at the corner portions was determined. Ratio R was determined by Equation 1 above, similar to the first test.

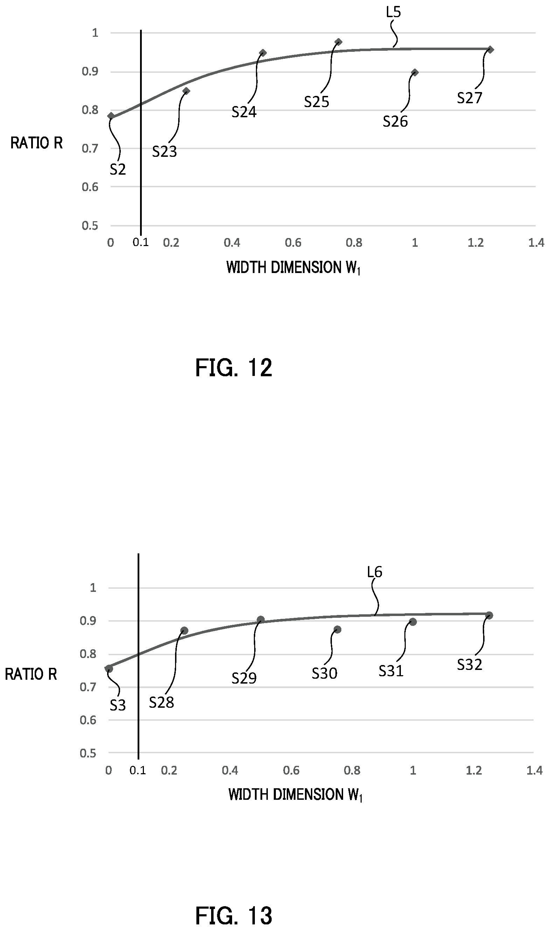

FIG. 12 is a diagram showing the measurement results of ratios R of test pieces S23 to S27 calculated based on the measurement results. In FIG. 12, the horizontal axis indicates width dimension W₁. In addition, in FIG. 12, the vertical axis indicates ratio R. FIG. 12 shows ratio R for each of test pieces S23 to S27. Reference numerals S23 to S27 shown in FIG. 12 indicate ratios R of test pieces S23 to S27. Note that, for the ratio R value for width dimension W₁ = 0, the value of test piece S2 not provided with a dummy pad was employed.

In FIG. 12, approximation line L5 shows an approximation line obtained by approximating ratios R of test pieces S23 to S27 with a quadratic curve. Note that FIG. 12 also shows the measurement result of ratio R for the test piece having width dimension W₁ of zero. The test piece having width dimension W₁ of zero is a test piece that does not include a dummy pad portion.

FIG. 13 is a diagram showing the measurement results of ratios R of test pieces S28 to S32 calculated based on the measurement results. In FIG. 13, the horizontal axis indicates width dimension W₁. In addition, in FIG. 13, the vertical axis indicates ratio R. FIG. 13 shows ratio R for each of test pieces S28 to S32. Reference numerals S28 to S32 shown in FIG. 13 indicate ratios R of test pieces S28 to S32. Note that, for the ratio R value for width dimension W₁ = 0, the value of test piece S3 not provided with a dummy pad was employed.

In FIG. 13, approximation line L6 indicates an approximation line obtained by approximating ratios R of test pieces S28 to S32 with a quadratic curve. Note that FIG. 13 also shows the measurement result of ratio R for the test piece having width dimension W₁ of zero. The test piece having width dimension W₁ of zero is a test piece that does not include a dummy pad portion.

As shown in FIGS. 12 and 13, ratios R of test pieces S23 to S27 and ratios R of test pieces S28 to S32 are 0.8 or more in a range in which width dimension W₁ is 0.10 mm or more. In addition, as shown in FIGS. 12 and 13, ratios R of test pieces S23 to S27 and ratios R of test pieces S28 to S32 tend to increase as width dimension W₁ increases.

In other words, the variation in the thickness dimension of sensing portions 34 tends to decrease as width dimension W₁ increases. When ratio R is 0.8 or more, the variation in the thickness dimension of sensing portions 34 is sufficiently small. Thus, width dimension W₁ is preferably 0.10 mm or more. Width dimension W₁ is more preferably 0.25 mm or more. Note that the upper limit of width dimension W₁ may be set as appropriate according to the size of substrate 3. For example, the upper limit of width dimension W₁ may be 1.25 mm.

### (Regarding Functions and Effects of Present Embodiment)

As described above, in the case of substrate 3 according to the present embodiment, substrate 3 includes dummy pad portion 36. Therefore, the variation in the thickness dimension of sensing portions 34 can be reduced. The reason for this is as described above. In addition, functions and effects obtained from the configuration of substrate 3 according to the present embodiment are as described above.

The disclosure of Japanese Patent Application No. 2023-212410, filed on December 15, 2023, including the specification, drawings and abstract, is incorporated herein by reference in its entirety.

### Industrial Applicability

The present invention can be suitably applied to various inspection apparatuses that measure a characteristic value of an object disposed on a substrate.

### Reference Signs List

1 Inspection system
2 Inspection apparatus
21 Apparatus main body
211 Housing
22 Substrate support portion
22R Inspection region
221, 222 Apparatus-side terminal portion
223 Mounting portion
224 Positioning member
225 Cover
23 Display portion
3, 3A, 3B, 3C, 3D, 3E, 3F, 3G Substrate
31 Substrate main body
31a Through-hole
311 First surface
312 Second surface
313 Through-hole
314 First metal layer
315 Second metal layer
316 Third metal layer
32, 32a, 32b, 32A, 32B, 32C1, 32C2, 32C3 Land portion
32D, 32E, 32F Land portion
33 Land base portion
331 Base main body
331a First base layer
331b Second base layer
331c Connecting portion
332 Resin portion
333 First cover layer
334 Second cover layer
34, 34A, 34B, 34C1, 34C2, 34C3, 34D, 34E, 34F Sensing portion
35 Terminal portion
36, 36D, 36E, 36F, 36G Dummy pad portion
361 Base portion
361a First base layer
361b First cover layer
362 Plating layer
37a Horizontal symbol
37b Vertical symbol
5 Target cell
6 Fifth intermediate work-size substrate
61 Pattern portion
R₁ First region
R₂ Second region

## Claims

1. A substrate, comprising:
a substrate main body;
a plurality of land portions that each include a sensing portion and a terminal portion and are supported by the substrate main body; and
a dummy pad portion provided in a certain region surrounding a first region in which the sensing portion is provided in the substrate main body, the certain region being referred to as a second region.

2. The substrate according to claim 1, wherein
the sensing portion is provided on a first surface of the substrate main body,
the terminal portion is provided on a second surface of the substrate main body, and
the plurality of land portions each further include a connecting portion disposed in a through-hole of the substrate main body, the connecting portion connecting the sensing portion and the terminal portion together.

3. The substrate according to claim 2, wherein
the dummy pad portion is provided only on the first surface.

4. The substrate according to claim 2, wherein
the sensing portion is formed of a plating layer formed by an electroless plating method on a surface of the connecting portion.

5. The substrate according to claim 1, wherein
the sensing portion, during use, comes into contact with an object to be measured for impedance, and
the terminal portion, during use, comes into contact with an apparatus-side terminal portion of a measurement apparatus for measuring the impedance of the object.

6. The substrate according to claim 1, wherein
the first region is a rectangular region,
the second region is a frame-shaped region surrounding the first region, and
the dummy pad portion is provided in a portion of the second region including at least four corners.

7. The substrate according to claim 6, wherein
the dummy pad portion has a rectangular frame shape extending along the second region.

8. The substrate according to claim 7, wherein
the dummy pad portion has a continuous rectangular frame shape or a discontinuous rectangular frame shape.

9. The substrate according to claim 1, wherein
an area occupancy rate, which is a proportion of the first region occupied by the sensing portion, is 17% or more and 100% or less.

10. The substrate according to claim 9, wherein
the area occupancy rate is 30% or more and 88% or less.

11. The substrate according to claim 1, wherein
a distance between the sensing portion provided on an outermost edge side in the first region and the dummy pad portion is 0.1 mm or more and 1.00 mm or less.

12. The substrate according to claim 1, wherein
a width dimension of the dummy pad portion is 0.1 mm or more.

13. The substrate according to claim 12, wherein
the width dimension of the dummy pad portion is 0.25 mm or more.

14. The substrate according to claim 1, wherein
the substrate main body includes, around the dummy pad portion, a symbol for designating the sensing portion to be used during inspection, and
the dummy pad portion is segmented corresponding to the symbol.

15. An inspection system, comprising:
the substrate according to claim 1; and
an inspection apparatus that includes a substrate support portion for supporting the substrate and measures impedance of a target cell placed on the sensing portion of the substrate.
